(19) Europäisches Patentamt
European Patent Office
Office européen des brevets

(11) EP 4 810 527 A1

(12) **EUROPEAN PATENT APPLICATION**
published in accordance with Art. 153(4) EPC

(43) Date of publication:
**23.09.2026 Bulletin 2026/39**

(21) Application number: **24902324.3**

(22) Date of filing: **11.10.2024**

(51) International Patent Classification (IPC):
***C08B 37/00*** *(2006.01)* ***A61K 131/00*** *(2006.01)*
***A61K 31/715*** *(2006.01)*

(86) International application number:
**PCT/CN2024/124057**

(87) International publication number:
**WO 2025/123898 (19.06.2025 Gazette 2025/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **12.12.2023 CN 202311696623**

(71) Applicant: **Shaanxi Haixiang Plateau Biotechnology Co., Ltd**
**Xi'an, Shaanxi 710026 (CN)**

(72) Inventors:
- **CAO, Wei**
  **Xi'an, Shaanxi 710026 (CN)**
- **ZHAO, Wenjing**
  **Xi'an, Shaanxi 710026 (CN)**
- **NONG, Qiuna**
  **Xi'an, Shaanxi 710026 (CN)**

(74) Representative: **Bayramoglu et al.**
**Mira Office**
**Kanuni Sultan Süleyman Boulevard 5387**
**Street Beytepe, floor 12, no:50**
**06800 Cankaya, Ankara (TR)**

# (54) HIPPOPHAE RHAMNOIDES PEEL POLYSACCHARIDES, AND PREPARATION METHOD THEREFOR AND USE THEREOF

(57) Disclosed are a polysaccharide from *Hippophae rhamnoides* fruit peel, and a preparation method therefor and use thereof, where the polysaccharide includes the following monosaccharide components: rhamnose, mannose, galactose, glucose, galacturonic acid, and arabinose. The preparation method includes defatting, extraction, concentration, and alcohol precipitation to obtain a water-soluble crude polysaccharide, followed by purification using a non-polar macroporous adsorption resin to obtain the polysaccharide from *Hippophae rhamnoides* fruit peel. The preparation method provides a high polysaccharide yield and high polysaccharide content. With the non-polar macroporous adsorption resin instead of the costly DEAE-cellulose and dextran gel, the preparation method achieves enrichment of active polysaccharides from *Hippophae rhamnoides* fruit peel, greatly increases the yield and purity of the polysaccharide, and reduces production costs, making the process suitable for industrial-scale production. The polysaccharide from *Hippophae rhamnoides* fruit peel exhibits significant anti-ulcerative colitis activity and is effective in ameliorating constipation, functional dyspepsia, and gut microbiota dysbiosis.



Processed by Luminess, 75001 PARIS (FR)

## Description

## TECHNICAL FIELD

The present application belongs to the technical field of traditional Chinese medicine extraction, and particularly relates to a polysaccharide from *Hippophae rhamnoides* fruit peel, and a preparation method therefor and use thereof.

## BACKGROUND TECHNOLOGY

*Hippophae rhamnoides* is a dried mature fruit of *Hippophae rhamnoides* Linn., a plant of the Elaeagnaceae family, and is a medicinal and edible homologous plant. A *Hippophae rhamnoides* fruit includes pulp (about 68%), seeds (about 23%), and peel (about 8%). Each portion has extremely high medicinal value and contains more than 100 bioactive substances. At present, research on *Hippophae rhamnoides* fruit is mainly focused on *Hippophae rhamnoides* pulp and *Hippophae rhamnoides* seeds. A large number of studies have reported that whole *Hippophae rhamnoides* fruit and seed meal are mainly used for extraction of *Hippophae rhamnoides* seed oil, and *Hippophae rhamnoides* pulp is mainly used for extraction of *Hippophae rhamnoides* flavonoid compounds. *Hippophae rhamnoides* oil has abundant chemical components and unique properties, and contains various unsaturated fatty acids, carotenoids, natural vitamin E, and other components, most of which are active components essential to the human body. *Hippophae rhamnoides* flavonoids have various physiological functions, such as anti-inflammatory effects, liver protection, obesity improvement, and hypoglycemic effects. However, *Hippophae rhamnoides* fruit peel has not been effectively developed and utilized, mainly due to insufficient studies on component analysis of *Hippophae rhamnoides* fruit peel, optimization of extraction and purification processes, and pharmacological activity evaluation.

It is found from previous studies conducted by the inventors that, in *Hippophae rhamnoides* fruit peel, a polysaccharide content is greater than a flavonoid content, and the flavonoid content is greater than an oil content. The polysaccharide content is abundant.

Polysaccharides are natural high-molecular-weight carbohydrates formed by connecting a series of monosaccharides through glycosidic bonds. However, different Chinese herbal medicines have different polysaccharide compositions, which differ in molecular weight, monosaccharide composition, glycosidic bonds, and biological functions. Therefore, effective extraction, separation, and purification of polysaccharides from animals, plants, and microorganisms are of great significance for development of therapeutic drugs and healthcare products.

Polysaccharides are commonly extracted by a water-extraction and alcohol-precipitation method, and crude polysaccharides can be obtained after collection of precipitates. However, during a polysaccharide extraction process, pigments, proteins, and some small-molecule compounds are also dissolved together with polysaccharides, and a series of impurity-removal and purification procedures are subsequently required.

A polysaccharide impurity-removal process mainly includes protein removal and pigment removal. Conventional methods for polysaccharide protein removal mainly include a repeated freeze-thaw method, a trichloroacetic acid (TCA) method, and a Sevag method. The repeated freeze-thaw method is a physical protein-removal method and results in less polysaccharide loss; however, this method is time-consuming and has low efficiency. The TCA method and the Sevag method operate on a principle that proteins undergo irreversible denaturation under the action of organic reagents, thereby precipitating from a solution, and proteins are removed through centrifugation. The TCA method and the Sevag method have high protein-removal efficiency; however, polysaccharide activity is easily damaged and organic reagent residues are easily generated. Conventional methods for polysaccharide decolorization mainly include an activated carbon method and an $H_2O_2$ method. The activated carbon method operates on a principle that impurities and pigments in a polysaccharide solution are adsorbed through a favorable adsorption performance of activated carbon, thereby decolorizing the polysaccharide solution; however, polysaccharides are easily mixed with activated carbon and are difficult to separate. The $H_2O_2$ method operates on a principle that pigment molecules in polysaccharide substances are oxidized into colorless substances through an oxidation process. The $H_2O_2$ method provides a favorable decolorization effect and high efficiency; however, a concentration and treatment time of hydrogen peroxide need to be controlled to avoid damage to polysaccharide molecules caused by oxidation.

A polysaccharide purification process is generally performed using techniques such as a stepwise precipitation method, an ion-exchange chromatography method, and a gel chromatography method. According to the stepwise precipitation method, due to differences in polysaccharide structure, molecular weight, polarity, and solubility in an organic solvent, a concentration of an organic reagent (such as methanol and ethanol) is continuously increased, such that polysaccharides precipitate sequentially from a largest molecular weight to a smallest molecular weight. However, it is generally necessary to obtain homogeneous polysaccharides. The ion-exchange chromatography method achieves separation according to differences in ion-exchange capacity or selectivity coefficient of target polysaccharides by using a chemically bonded ion exchanger or an ion-exchange resin as a stationary phase. A DEAE-cellulose column is commonly used to achieve favorable separation of neutral polysaccharides and acidic polysaccharides. This is because acidic

polysaccharides contain anions and can be adsorbed by a chromatographic column, and neutral polysaccharides flow out of the chromatographic column first, thereby purifying polysaccharides. The gel chromatography method performs separation by using an exclusion effect of a porous gel on molecules of different sizes, thereby achieving efficient separation of macromolecular substances. Cross-linked dextran gel Sephadex and agarose gel Sepharose are commonly used for separation and purification of polysaccharides. A DEAE-cellulose column and a gel column can provide favorable separation effects for polysaccharide substances. However, chromatographic separation is relatively slow due to small filler pores, filler materials are expensive, and these columns are suitable for laboratory applications, thereby resulting in certain limitations.

In recent years, macroporous resin adsorption technology has been applied to extraction and purification of natural products, and has been widely used for flavonoids, alkaloids, and saponins. A macroporous resin adsorption method selectively adsorbs substances from a solution through physical adsorption according to differences in molecular weight and structure of substances, thereby purifying target substances. In theory, purification of polysaccharides can be achieved using macroporous adsorption resins. In this way, purification time can be greatly shortened and a process flow can be simplified. However, adsorption and desorption capacities of macroporous resins for most polysaccharides are weak, separation effects are poor, and few studies have investigated purification of polysaccharides using such a method. Accordingly, screening of suitable resins according to properties of polysaccharides is relatively complex and difficult.

According to a search, DEAE-cellulose columns and dextran gel columns are currently commonly used for purification and enrichment of crude polysaccharide from *Hippophae rhamnoides* to obtain pharmacologically active polysaccharide from *Hippophae rhamnoides*. Chinese Patent Application No. CN115028753A discloses a homogeneous *Hippophae rhamnoides* polysaccharide having an antitumor effect, and a separation and purification method therefor and use thereof. A crude powder of dried *Hippophae rhamnoides* fruits is extracted using a flash extraction method; an extract is subsequently subjected to impurity removal through precipitation with absolute ethanol, protein removal treatment using a Sevag reagent, and decolorization treatment using an $H_2O_2$ solution to obtain a refined polysaccharide from *Hippophae rhamnoides*; and the polysaccharide from *Hippophae rhamnoides* is then purified using a DEAE-52 cellulose column and a Sephadex G-200 chromatographic column to obtain a homogeneous *Hippophae rhamnoides* polysaccharide having an antitumor effect, with a yield of 16.61% and a sugar content of 58.43 mg/g. Chinese Patent Application No. CN110790848A discloses a preparation method of a total polysaccharide from *Hippophae rhamnoides* and use thereof. Defatted *Hippophae rhamnoides* fruits are extracted using distilled water, followed by precipitation with absolute ethanol and centrifugation; a polysaccharide precipitate is dried at 40 °C to prepare a water-soluble crude polysaccharide, and protein impurities are removed from the crude polysaccharide using a Sevag method; and subsequently, separation and purification are performed using ion-exchange resin DEAE-52 and dextran gel Sephadex G-150 to obtain the total polysaccharide from *Hippophae rhamnoides* having antioxidant and anti-fatigue functions. A yield of the total polysaccharides *Hippophae rhamnoides* obtained by the extraction method is 10.61%. In addition, Chinese Patent Application No. CN103992299A discloses a method for simultaneously separating and purifying bioactive components from *Hippophae rhamnoides* seed meal. An extract of *Hippophae rhamnoides* seed meal is eluted using AB-8 macroporous adsorption resin, and a polysaccharide from *Hippophae rhamnoides* is collected with a purity greater than 80%.

According to Chinese Patent Application No. CN103992299A, the AB-8 macroporous resin used is a medium-polarity macroporous adsorption resin and is suitable for separation of polysaccharide components obtained after alcohol extraction of *Hippophae rhamnoides* seed meal. A monosaccharide composition and pharmacological activity of the *Hippophae rhamnoides* seed meal polysaccharide are unknown.

At present, although numerous studies have been conducted on a polysaccharide from *Hippophae rhamnoides*, polysaccharide sources are different. Most polysaccharides are derived from *Hippophae rhamnoides* fruits, and few polysaccharides are derived from *Hippophae rhamnoides* fruit peel. Polysaccharides from different sources have different monosaccharide compositions, sugar residues, and molecular weights, and pharmacological activities of such polysaccharides differ significantly. In addition, DEAE-cellulose columns and dextran gel columns commonly used during purification of polysaccharides from *Hippophae rhamnoides* are expensive, the chromatographic separation rates are slow, the yields and purities of obtained polysaccharides from *Hippophae rhamnoides* remain relatively low, and the functional evaluations of polysaccharides from *Hippophae rhamnoides* are relatively limited. Therefore, there is still a need to further study a polysaccharide from *Hippophae rhamnoides* fruit peel, and a preparation method thereof and use thereof.

## CONTENT OF THE INVENTION

Aiming at the defects in the prior art, the present application provides a polysaccharide from *Hippophae rhamnoides* fruit peel, and a preparation method therefor and use thereof. A DEAE-cellulose column and a dextran gel column are replaced with a non-polar macroporous adsorption resin to treat a crude polysaccharide obtained from *Hippophae rhamnoides* fruit peel, thereby achieving enrichment of the polysaccharide from *Hippophae rhamnoides* fruit peel while removing most impurities. The preparation method of the present application effectively improves a yield and a

purity of the polysaccharide from *Hippophae rhamnoides*, maintain a structure and biological activity of the polysaccharide without organic solvent residues, and is suitable for large-scale preparation of the polysaccharide from *Hippophae rhamnoides* fruit peel.

The inventors have found that the polysaccharide from *Hippophae rhamnoides* fruit peel of the present application has a significant effect of treating ulcerative colitis. A low-dose treatment group (50 mg/kg/d of *polysaccharide* from Hippophae rhamnoides fruit peel) may improve ulcerative colitis. A high-dose treatment group (100 mg/kg/d of polysaccharide from *Hippophae rhamnoides* fruit peel) can significantly increase a body weight and a colon length of dextran sulfate sodium (DSS)-induced ulcerative colitis (UC) mice, reduce a disease activity index, significantly inhibit increases of pro-inflammatory factors in serum and colon tissue of DSS-induced UC mice, promote secretion of anti-inflammatory factors, effectively inhibit an increase in intestinal permeability of DSS-induced UC mice, and significantly reduce colon tissue damage and restore intestinal barrier function of colon tissue. Compared with a polysaccharide from *Hippophae rhamnoides* fruits reported in the prior art that exerts an ulcerative colitis therapeutic effect at a dose of 200 mg/kg/d [Wang Xinxu, *Regulatory Mechanism of Seabuckthorn Polysaccharide on DSS-Induced Ulcerative Colitis in Mice* [D], Inner Mongolia Agricultural University, 2019], the polysaccharide from *Hippophae rhamnoides* fruit peel provided in the present application achieves a favorable therapeutic effect at a dose of 100 mg/kg/d, and exhibits superior efficacy relative to the above polysaccharide from *Hippophae rhamnoides* fruits.

In addition, the inventors have found that the polysaccharide from *Hippophae rhamnoides* fruit peel of the present application has a significant digestion-promoting function, improves functional constipation and dyspepsia, and significantly improves gut microbiota dysbiosis.

A first objective of the present application is to provide a polysaccharide from *Hippophae rhamnoides* fruit peel.

The polysaccharide from *Hippophae rhamnoides* fruit peel provided by the present application includes the following monosaccharide components: rhamnose, mannose, galactose, glucose, galacturonic acid, and arabinose, where a molar ratio of the rhamnose, the mannose, the galactose, the glucose, the galacturonic acid, and the arabinose is 1:2.03-2.63:2.65-3.17:3.20-3.97:4.01-7.33:7.95-11.67.

Preferably, the molar ratio of the rhamnose, the mannose, the galactose, the glucose, the galacturonic acid, and the arabinose is 1:2.10-2.63:2.65-3.17:3.20-3.97:4.10-6.35:9.60-11.30.

Preferably, a mass percentage content of neutral sugars in the polysaccharide from *Hippophae rhamnoides* fruit peel is 82.73%-85.31%.

Preferably, a molecular weight distribution range of the polysaccharide from *Hippophae rhamnoides* fruit peel is 2.5 kDa to $2.5 \times 10^2$ kDa.

Preferably, a mass percentage content of uronic acid in the polysaccharide from *Hippophae rhamnoides* fruit peel is 16.90%-23.78%.

Preferably, the polysaccharide from *Hippophae rhamnoides* fruit peel is a yellow amorphous powder having a fine texture, is soluble in water, and exhibits a yellow color upon reaction with a phenol-sulfuric acid reagent.

A second objective of the present application is to provide a preparation method for a polysaccharide from *Hippophae rhamnoides* fruit peel, where the preparation method can obtain the polysaccharide from *Hippophae rhamnoides* fruit peel on a large scale.

The preparation method for the polysaccharide from *Hippophae rhamnoides* fruit peel includes the following steps:

(1) defatting: adding dried *Hippophae rhamnoides* fruit peel to an organic solvent for extraction by a reflux method, a maceration method, or a percolation method, discarding an extract, and obtaining defatted *Hippophae rhamnoides* fruit peel residue;
(2) extraction: drying the defatted *Hippophae rhamnoides* fruit peel residue, soaking the defatted *Hippophae rhamnoides* fruit peel residue in water, subsequently performing reflux extraction, and filtering to obtain an extract solution;
(3) concentration: concentrating the extract solution and cooling to obtain a concentrated solution;
(4) alcohol precipitation: adding absolute ethanol to the concentrated solution, or adding the concentrated solution to a high-concentration aqueous ethanol solution, allowing the resulting mixture to stand, and collecting a precipitate to obtain a crude polysaccharide from *Hippophae rhamnoides* fruit peel; and
(5) purification: dissolving the crude polysaccharide from *Hippophae rhamnoides* fruit peel in water, adding the resulting solution to non-polar macroporous adsorption resin D101, D312, X-5, or HP-20, performing elution, and concentrating and drying an eluate to obtain the polysaccharide from *Hippophae rhamnoides* fruit peel.

Preferably, the preparation method for the polysaccharide from *Hippophae rhamnoides* fruit peel includes the following steps:

(1) defatting: drying *Hippophae rhamnoides* fruit peel, adding an organic solvent at a solid-to-liquid ratio of 1:5-10

(kg/L) for reflux extraction, where the extraction is performed for 2-3 h 1-3 times, filtering, and discarding an extract solution to obtain defatted *Hippophae rhamnoides* fruit peel residue;

(2) extraction: drying the defatted *Hippophae rhamnoides* fruit peel residue, adding water (e.g., distilled water) at a solid-to-liquid ratio of 1:6-10 (kg/L) to soak the residue for several hours, subsequently performing reflux extraction, where the extraction is performed for 2-3 h 2-3 times, filtering, and combining extract solutions;

(3) concentration: concentrating the extract solutions to 1/50-1/4 of an original volume and cooling to room temperature to obtain a concentrated solution;

(4) alcohol precipitation: adding absolute ethanol to the concentrated solution under stirring such that a volume concentration of ethanol is 60%-80%, or adding the concentrated solution to a high-concentration aqueous ethanol solution such that a mass percentage content of ethanol in the concentrated solution is 60%-80%, allowing the resulting mixture to stand at 4-25 °C for 6-24 h, discarding a supernatant, and collecting a precipitate to obtain a crude polysaccharide from *Hippophae rhamnoides* fruit peel; and

(5) purification: performing decolorization and protein-removal treatment on the crude polysaccharide from *Hippophae rhamnoides* fruit peel using non-polar macroporous adsorption resin D101, D312, X-5, or HP-20; dissolving the crude polysaccharide from *Hippophae rhamnoides* fruit peel in water, slowly adding a resulting solution to the non-polar macroporous adsorption resin, eluting with water, collecting an eluate, concentrating the eluate to 1/80-1/2 of an original volume, and drying to obtain the polysaccharide from *Hippophae rhamnoides* fruit peel.

Preferably, in step (1), the organic solvent is ether, petroleum ether, ethyl acetate, an aqueous methanol solution containing 75% to 100% methanol by mass, or an aqueous ethanol solution containing 75% to 100% ethanol by mass.

Preferably, in step (2), a soaking time after addition of water (e.g., tap water) to the defatted *Hippophae rhamnoides* fruit peel residue is 3-24 h.

Preferably, in step (4), the high-concentration aqueous ethanol solution contains 80% to 100% ethanol by mass.

Preferably, in step (5), the non-polar macroporous adsorption resin is D101 macroporous adsorption resin.

Preferably, in step (5), a mass concentration of a loading solution of the crude polysaccharide from *Hippophae rhamnoides* fruit peel is 0.005-0.03 kg/L, a loading amount is 0.01-0.15 kg/kg (polysaccharide/resin), a loading flow rate is 1-5 bed volumes (BV)/h, and standing is performed for 12-24 h after completion of loading.

Preferably, in step (5), the elution is performed using water (e.g., distilled water), an elution flow rate is 1-5 BV/h, and an elution volume is 1-10 BV.

Preferably, in step (5), the drying is spray drying, reduced-pressure drying, or freeze drying.

Preferably, in step (5), a decolorization rate is greater than 75%, a protein removal rate is greater than 73%, and a polysaccharide retention rate is greater than 70%.

A third objective of the present application is to provide use of the polysaccharide from *Hippophae rhamnoides* fruit peel in preparation of a medicament or a functional health product for treating an intestinal functional disease.

Preferably, the intestinal functional disease includes one or more of ulcerative colitis, constipation, dyspepsia, and gut microbiota dysbiosis.

Preferably, the polysaccharide from *Hippophae rhamnoides* fruit peel exerts an anti-inflammatory effect through promoting secretion of anti-inflammatory factors.

In addition, the present application provides a composition including the polysaccharide from *Hippophae rhamnoides* fruit peel or the polysaccharide from *Hippophae rhamnoides* fruit peel prepared by the preparation method, and a pharmaceutically acceptable excipient.

The composition, medicament, or functional health product of the present application is prepared according to a corresponding conventional pharmaceutical formulation method. The composition, medicament, or functional health product of the present application uses the polysaccharide from *Hippophae rhamnoides* fruit peel as a sole active ingredient or as one of active ingredients. Those skilled in the art may add suitable auxiliary ingredients according to actual requirements and prepare a corresponding dosage form using conventional technical means. This is not particularly limited in the present application.

The present application provides the following beneficial effects. A polysaccharide from *Hippophae rhamnoides* fruit peel, and a preparation method therefor and use thereof are provided. In the preparation method, a DEAE-cellulose column and a dextran gel column are replaced with a non-polar macroporous adsorption resin for enrichment and purification of a crude polysaccharide from *Hippophae rhamnoides* fruit peel, thereby obtaining a polysaccharide from *Hippophae rhamnoides* fruit peel having high application value. A yield of the preparation method for polysaccharide provided in the present application can reach greater than 18.86%, and a neutral sugar content in the polysaccharide can reach greater than 82.73%. Compared with existing methods, this preparation method greatly improves polysaccharide purity and yield. The preparation method has a simple operation process, and the obtained polysaccharide has high purity. The industrial production costs can be greatly reduced, residual organic solvents generated during operation can be avoided, and the green development principles can be satisfied.

## SPECIFIC IMPLEMENTATIONS

The typical examples are provided below to further illustrate the present application and do not constitute limitations on the present application in any form.

Unless otherwise specified, all contents described below are mass percentages.

Unless otherwise specified, an ethanol solution described below is an aqueous ethanol solution

### Example 1

600 kg of dried *Hippophae rhamnoides* fruit peel was weighed and 4000 L of petroleum ether was added for maceration extraction, where the extraction was performed for 12 h. After completion of extraction, a petroleum ether extract solution was filtered off, and *Hippophae rhamnoides* fruit peel residue was retained. Petroleum ether remaining in the defatted *Hippophae rhamnoides* fruit peel residue was evaporated to dryness, and 4000 L of water was added to soak the residue for 6 h. Reflux extraction was subsequently performed three times for 2 h each time, followed by filtration. Water extracts were combined, and an extract solution was introduced into a double-effect concentrator for concentration under reduced pressure until a final volume of 1200 L was obtained. After cooling to room temperature, 3 volumes of absolute ethanol were added to the concentrated extract solution. The resulting mixture was allowed to stand at 15 °C for 24 h, and a precipitate was collected. The precipitate was dissolved in water at a concentration of 0.01 kg/L. The resulting solution was loaded onto D312 macroporous adsorption resin at a loading amount of 0.05 kg/kg (polysaccharide/resin), where a loading flow rate was controlled at 3 BV/h. After completion of loading, the resin was allowed to stand for 12 h. Elution was subsequently performed by water, where an elution flow rate was controlled at 3 BV/h, and an elution volume was 6 BV. After completion of elution, an eluate was collected, and an extract solution was introduced into a double-effect concentrator for concentration under reduced pressure until a final volume of 1800 L was obtained. The concentrated solution was dried under reduced pressure to obtain a polysaccharide from *Hippophae rhamnoides* fruit peel, where a neutral sugar content of the polysaccharide was 82.75%.

### Example 2

800 kg of dried *Hippophae rhamnoides* fruit peel was weighed, and 5500 L of 85% aqueous methanol solution was added for defatting treatment. Reflux extraction was performed for 3 h. After completion of extraction, a methanol extract solution was filtered off, and *Hippophae rhamnoides* fruit peel residue was retained. The *Hippophae rhamnoides* fruit peel residue was further treated once according to the defatting method described above, and a methanol extract solution was filtered off after completion of extraction. Methanol remaining in the defatted *Hippophae rhamnoides* fruit peel residue was evaporated to dryness, and 5500 L of water was added to soak the residue for 12 h. Reflux extraction was subsequently performed three times for 2 h each time, followed by filtration. Water extracts were combined, and an extract solution was introduced into a double-effect concentrator for concentration under reduced pressure until a final volume of 2000 L was obtained. After cooling to room temperature, 3 volumes of absolute ethanol were added to the concentrated extract solution. The resulting mixture was allowed to stand at 4 °C for 12 h, and a precipitate was collected. The precipitate was dissolved in water at a concentration of 0.015 kg/L. The resulting solution was loaded onto HP-20 macroporous adsorption resin at a loading amount of 0.03 kg/kg (polysaccharide/resin), where a loading flow rate was controlled at 4 BV/h. After completion of loading, the resin was allowed to stand for 18 h. Elution was subsequently performed by water, where an elution flow rate was controlled at 4 BV/h, and an elution volume was 5 BV. After completion of elution, an eluate was collected, and an extract solution was introduced into a double-effect concentrator for concentration under reduced pressure until a final volume of 2200 L was obtained. The concentrated solution was freeze-dried to obtain a polysaccharide from *Hippophae rhamnoides* fruit peel, where a neutral sugar content of the polysaccharide was 83.67%.

### Example 3

500 kg of dried *Hippophae rhamnoides* fruit peel was weighed, and 3000 L of ethyl acetate was added for percolation treatment, where a treatment time was 12 h. After completion of treatment, an ethyl acetate extract solution was filtered off to obtain defatted *Hippophae rhamnoides* fruit peel residue. Ethyl acetate remaining in the defatted *Hippophae rhamnoides* fruit peel residue was evaporated at 50 °C, and 4000 L of water was added to soak the residue for 6 h. Reflux extraction was subsequently performed three times for 2 h each time, followed by filtration. Water extracts were combined, and an extract solution was introduced into a double-effect concentrator for concentration under reduced pressure until a final volume of 800 L was obtained. After cooling to room temperature, the concentrated extract solution was added into 3200 L of 85% aqueous ethanol solution. The resulting mixture was allowed to stand at 20 °C for 20 h, and a precipitate was collected. The precipitate was dissolved in water at a concentration of 0.01 kg/L. The resulting solution was loaded onto X-5 macroporous adsorption resin at a loading amount of 0.12 kg/kg (polysaccharide/resin), where a loading

flow rate was controlled at 4 BV/h. After completion of loading, the resin was allowed to stand for 12 h. Elution was subsequently performed by water, where an elution flow rate was controlled at 3 BV/h, and an elution volume was 6 BV. After completion of elution, an eluate was collected, and an extract solution was introduced into a double-effect concentrator for concentration under reduced pressure until a final volume of 1900 L was obtained. The concentrated solution was spray-dried to obtain a polysaccharide from *Hippophae rhamnoides* fruit peel, where a neutral sugar content of the polysaccharide was 82.73%.

**Example 4**

600 kg of dried *Hippophae rhamnoides* fruit peel was weighed, 4000 L of 80% aqueous ethanol solution was added for reflux extraction, and *Hippophae rhamnoides* fruit peel residue was defatted, where the extraction was performed for 3 h. After completion of extraction, an ethanol extract solution was filtered off, and the *Hippophae rhamnoides* fruit peel residue was retained. The *Hippophae rhamnoides* fruit peel residue was further treated once according to the extraction method described above, and an ethanol extract solution was filtered off after completion of extraction. Ethanol remaining in the defatted *Hippophae rhamnoides* fruit peel residue was evaporated at 60 °C, and 4000 L of water was added to soak the residue for 3 h. Reflux extraction was subsequently performed three times for 2 h each time, followed by filtration. Water extracts were combined, and an extract solution was introduced into a double-effect concentrator for concentration under reduced pressure until a final volume of 1000 L was obtained. After pre-cooling at 10 °C for 6 h, the concentrated extract solution was added into 3000 L of 88% aqueous ethanol solution. The resulting mixture was allowed to stand at 25 °C for 22 h, and a precipitate was collected. The precipitate was dissolved in water at a concentration of 0.025 kg/L. The resulting solution was loaded onto D101 macroporous adsorption resin at a loading amount of 0.15 kg/kg (polysaccharide/resin), where a loading flow rate was controlled at 2 BV/h. After completion of loading, the resin was allowed to stand for 12 h. Elution was subsequently performed by water, where an elution flow rate was controlled at 3 BV/h, and an elution volume was 5 BV. After completion of elution, an eluate was collected, and an extract solution was introduced into a double-effect concentrator for concentration under reduced pressure until a final volume of 1500 L was obtained. The concentrated solution was freeze-dried to obtain a polysaccharide from *Hippophae rhamnoides* fruit peel, where a neutral sugar content of the polysaccharide was 85.31%.

**Example 5**

900 kg of dried *Hippophae rhamnoides* fruit peel was weighed, 5500 L of 75% aqueous ethanol solution was added for reflux extraction, and *Hippophae rhamnoides* fruit peel residue was defatted, where the extraction time is performed for 3 h. After completion of extraction, an ethanol extract solution was filtered off, and *Hippophae rhamnoides* fruit peel residue was retained. The *Hippophae rhamnoides* fruit peel residue was further treated once according to the extraction method described above, and an ethanol extract solution was filtered off after completion of extraction. Ethanol remaining in the defatted *Hippophae rhamnoides* fruit peel residue was evaporated to dryness at 50 °C, and 5500 L of water was added to soak the residue for 5 h. Reflux extraction was subsequently performed twice for 3 h each time, followed by filtration. Water extracts were combined, and an extract solution was introduced into a double-effect concentrator for concentration under reduced pressure until a final volume of 950 L was obtained. After cooling to room temperature, 2 volumes of absolute ethanol were added to the concentrated extract solution. The resulting mixture was allowed to stand at 4 °C for 12 h, and a precipitate was collected. The precipitate was dissolved in water at a concentration of 0.015 kg/L. The resulting solution was loaded onto D101 macroporous adsorption resin at a loading amount of 0.05 kg/kg (polysaccharide/resin), where a loading flow rate was controlled at 4 BV/h. After completion of loading, the resin was allowed to stand for 12 h. Elution was subsequently performed by water, where an elution flow rate was controlled at 3 BV/h, and an elution volume was 6 BV. After completion of elution, an eluate was collected, and an extract solution was introduced into a double-effect concentrator for concentration under reduced pressure until a final volume of 3000 L was obtained. The concentrated solution was spray-dried to obtain a polysaccharide from *Hippophae rhamnoides* fruit peel, where a neutral sugar content of the polysaccharide was 84.58%.

**Example 6**

600 kg of dried *Hippophae rhamnoides* fruit peel was weighed, and 3000 L of 95% aqueous ethanol solution was added for reflux extraction for 2 h. After completion of extraction, an ethanol extract solution was filtered off, and *Hippophae rhamnoides* fruit peel residue was retained. The *Hippophae rhamnoides* fruit peel residue was further treated once according to the extraction method described above, and an ethanol extract solution was filtered off after completion of extraction. Ethanol remaining in the defatted *Hippophae rhamnoides* fruit peel residue was evaporated to dryness, and 3600 L of water was added to soak the residue for 10 h. Reflux extraction was subsequently performed twice for 3 h each time, followed by filtration. Water extracts were combined, and an extract solution was introduced into a double-effect

concentrator for concentration under reduced pressure until a final volume of 1700 L was obtained. After cooling to room temperature, the concentrated extract solution was added into 5500 L of 80% aqueous ethanol solution. The resulting mixture was allowed to stand at 4 °C for 24 h, and a precipitate was collected. The precipitate was dissolved in water at a concentration of 0.03 kg/L. The resulting solution was loaded onto D312 macroporous adsorption resin at a loading amount of 0.12 kg/kg (polysaccharide/resin), where a loading flow rate was controlled at 4 BV/h. After completion of loading, the resin was allowed to stand for 24 h. Elution was subsequently performed by water, where an elution flow rate was controlled at 2 BV/h, and an elution volume was 4 BV. After completion of elution, an eluate was collected, and an extract solution was introduced into a double-effect concentrator for concentration under reduced pressure until a final volume of 1500 L was obtained. The concentrated solution was freeze-dried to obtain a polysaccharide from *Hippophae rhamnoides* fruit peel, where a neutral sugar content of the polysaccharide was 83.95%.

**Comparative Example 1**

A polysaccharide from *Hippophae rhamnoides* seed meal [A method for simultaneously separating and purifying bioactive components from *Hippophae rhamnoides* seed meal, Chinese Patent Application Publication No. CN103992299A] was prepared according to the following steps:

0.5 kg of *Hippophae rhamnoides* seed meal obtained after supercritical $CO_2$ extraction of *Hippophae rhamnoides* seed oil was added to 5 L of 75% aqueous ethanol solution; 25 g of cellulase and 10 g of pectinase were added, and extraction was performed three times under 500 W ultrasonic conditions, where a pH value was controlled at 3.0, a temperature was controlled at 40 °C, and the extraction was performed once for 2 h; three extraction solutions were combined, ethanol was recovered by rotary evaporation, an aqueous solution was obtained, and the aqueous solution was filtered through a 200-mesh filter cloth to obtain a filtrate; the filtrate was adsorbed using AB-8 macroporous resin and eluted with water until colorless, and an eluate was collected to obtain a crude polysaccharide; and the crude polysaccharide was freeze-dried and pre-frozen at -50 °C for 2 h, followed by vacuum drying for 24 h to obtain a powdery polysaccharide from *Hippophae rhamnoides* seed meal. A neutral sugar content of the polysaccharide was determined to be 83.1%.

**Comparative Example 2**

A polysaccharide from *Hippophae rhamnoides* fruits [Wang Xinxu, Regulatory Mechanism of Seabuckthorn Polysaccharide on DSS-Induced Ulcerative Colitis in Mice[D], Inner Mongolia Agricultural University, 2019.] was prepared according to the following steps:

100 g of dried *Hippophae rhamnoides* fruit was ground in a mortar and passed through a 30-mesh sieve. 800 mL of petroleum ether was added for defatting treatment, and a filtrate was recovered. Distilled water was added, and ultrasonic disruption was performed, followed by hot-water extraction. A water extract solution was concentrated under reduced pressure at 80 °C. Sevag reagent was added for deproteinization. Absolute ethanol was added to the deproteinized sample solution in a beaker until a final concentration of 80%. The resulting mixture was stored in a refrigerator at 4 °C for alcohol precipitation. After 48 h, a precipitate was formed to obtain a crude polysaccharide. The crude polysaccharide was dissolved in distilled water to prepare a 5% polysaccharide solution. Concentrated ammonia water was used to adjust pH to approximately 8.0. $H_2O_2$ was added dropwise below 50 °C until a light yellow color was observed, and the mixture was incubated for 2 h. The solution was placed in a dialysis membrane (with a molecular weight cutoff of 8000-14000), dialyzed with running water for 72 h and distilled water for 48 h, followed by freeze-drying to obtain a polysaccharide from *Hippophae rhamnoides* fruits. A neutral sugar content of the polysaccharide was 60.01%.

The following test examples were provided to further illustrate the physicochemical properties of the polysaccharide from *Hippophae rhamnoides* fruit peel, the evaluation of purification effects of the polysaccharide preparation method using non-polar macroporous adsorption resins (D101, D312, X-5, HP-20), and the therapeutic effects of the polysaccharide on intestinal functional diseases.

**Test Example 1: Determination of Neutral Sugar, Protein, and Uronic Acid Contents of Polysaccharide from *Hippophae Rhamnoides* Fruit Peel**

Determination of neutral sugar content: The neutral sugar content was determined by the phenol-sulfuric acid method. Glucose standard solutions at concentrations of 0.00, 0.02, 0.04, 0.06, 0.08, 0.10, and 0.12 mg/mL were prepared to construct a standard curve. Polysaccharides obtained in Examples 1-6 were accurately weighed and prepared into 0.1 mg/mL solutions. 2 mL of each sample solution was accurately transferred into a test tube, and 2 mL of 6% phenol and 6 mL of concentrated $H_2SO_4$ were sequentially added, followed by mixing. The mixture was allowed to stand for 10 min and then heated in a 100 °C water bath for 20 min. After cooling to room temperature, absorbance was measured at 490 nm. According to the standard curve, neutral sugar contents of the polysaccharides were calculated as 82.75%, 83.67%, 82.73%, 85.31%, 84.58%, and 83.95%, respectively.

Determination of Protein content: Protein content was determined using the Coomassie brilliant blue method. Bovine serum albumin standard solutions at concentrations of 0.00, 0.02, 0.04, 0.06, 0.08, 0.10, and 0.12 mg/mL were prepared to construct a standard curve. Polysaccharides obtained in Examples 1-6 were accurately weighed and prepared into 0.2 mg/mL solutions. 3 mL of each sample solution was accurately transferred into a test tube, and 3 mL of Coomassie brilliant blue reagent was added and mixed uniformly. Absorbance was measured at 595 nm. According to the standard curve, protein contents were calculated as 2.54%, 2.24%, 2.33%, 2.11%, 2.17%, and 2.19%, respectively.

Determination of uronic acid content: Uronic acid content was determined using the sulfuric acid-carbazole method. Galacturonic acid standard solutions at concentrations of 0.00, 0.02, 0.04, 0.06, 0.08, 0.10, and 0.12 mg/mL were prepared to construct a standard curve. Polysaccharides obtained in Examples 1-6 were accurately weighed and prepared into 0.1 mg/mL solutions. 2 mL of each sample solution was accurately transferred into a test tube, and 6 mL of concentrated $H_2SO_4$ was added and mixed uniformly, followed by cooling to room temperature. Subsequently, 1 mL of 0.15% carbazole solution was added and mixed uniformly, and the mixture was allowed to stand for 30 min. Absorbance was measured at 530 nm. According to the standard curve, uronic acid contents were calculated as 21.72%, 22.50%, 23.78%, 16.90%, 16.95%, and 17.53%, respectively.

## Test Example 2: Monosaccharide Composition Analysis of Polysaccharide from *Hippophae Rhamnoides* Fruit Peel

PMP derivatization: Standard solutions of Man, Rha, GlcA, GalA, Glc, Gal, Ara, and Fuc at a concentration of 10 mg/mL were prepared. 400 μL of each standard solution was transferred into one of eight 50 mL round-bottom flasks. Subsequently, 200 μL of 0.3 mol/L NaOH solution and 400 μL of 0.5 mol/L PMP methanol solution were sequentially added. The mixture was mixed uniformly, purged with nitrogen for 5 min, and incubated in a 70 °C water bath for 2 h. After cooling to room temperature, pH was adjusted to 7 using 0.3 mol/L HCl solution. Subsequently, 2 mL of $H_2O$ and 4 mL of $CHCl_3$ were sequentially added for extraction, an aqueous phase was retained, and $CHCl_3$ was added for extraction three additional times. Aqueous-phase derivatized samples were collected and filtered through a 0.45 μm aqueous membrane filter for HPLC analysis.

Complete acid hydrolysis of polysaccharide from *Hippophae rhamnoides* fruit peel: 10 mg of polysaccharide obtained in each of Examples 1-6 was accurately weighed and prepared into a 0.2 mg/mL solution. 1 mL of 2 mol/L trifluoroacetic acid solution was added. The resulting mixture was purged with nitrogen for 5 min, sealed, and heated in a 100 °C water bath for 8 h until complete acid hydrolysis of the polysaccharide was achieved. The the acid-hydrolyzed sample was cooled to room temperature and centrifuged at 4000 r/min for 5 min. A supernatant was collected. 5 mL of methanol was added to the supernatant, followed by rotary evaporation to dryness. The procedure was repeated 2-3 times to remove excess trifluoroacetic acid. Subsequently, 2 mL of distilled water was added, and pH was adjusted to 7 using 2 mol/L NaOH solution to obtain a complete acid hydrolysis product of the polysaccharide from *Hippophae rhamnoides* fruit peel.

PMP derivatization of complete acid hydrolysis products of polysaccharide from *Hippophae rhamnoides* fruit peel: 400 μL of each complete acid hydrolysis sample of the polysaccharide from *Hippophae rhamnoides* fruit peel was subjected to PMP derivatization according to the procedure used for the standards.

HPLC conditions: A DIONEX UltiMate chromatographic system was used. Chromatographic column: Accliam™ 120 C18 (4.6 mm i.d. × 250 mm, 5 μm, Thermo Fisher); flow rate: 1.0 mL/min; injection volume: 10 μL; column temperature: 30 °C; detection wavelength: 250 nm; and mobile phase:100 mmol/L ammonium acetate aqueous solution (pH 5.0):tetrahydrofuran:acetonitrile = 81:2:17.

According to monosaccharide retention times and peak areas, monosaccharide compositions and molar ratios of the polysaccharide from *Hippophae rhamnoides* fruit peel obtained in Examples 1-6 were determined as follows:

Example 1. rhamnose:mannose:galactose:glucose:galacturonic acid:arabinose = 1:2.13:2.71:3.30:4.22:9.65.
Example 2. rhamnose:mannose:galactose:glucose:galacturonic acid:arabinose = 1:2.42:2.69:3.67:5.25:9.88.
Example 3. rhamnose:mannose:galactose:glucose:galacturonic acid:arabinose = 1:2.35:2.74:3.81:6.25:10.13.
Example 4. rhamnose:mannose:galactose:glucose:galacturonic acid:arabinose = 1:2.55:3.05:3.86:6.31:11.24.
Example 5. rhamnose:mannose:galactose:glucose:galacturonic acid:arabinose = 1:2.62:3.14:3.96:4.82:10.56.
Example 6. rhamnose:mannose:galactose:glucose:galacturonic acid:arabinose = 1:2.59:3.07:3.93:4.17:9.81.

## Test Example 3: Determination of Molecular Weight Distribution Ranges of Polysaccharide from *Hippophae Rhamnoides* Fruit Peel

Chromatographic column: TSK-Gel G3000; mobile phase: 0.1 mol/L sodium sulfate solution; column temperature: 35 °C; flow rate: 0.5 mL/min; and detector: refractive index detector. Dextran standards (180, 2700, 5250, 9750, 13050, 36800, and 64650 Da) were used to prepare standard solutions, and a standard curve was drawn. Polysaccharides

obtained in Examples 1-6 were weighed and dissolved in 400 μL of the mobile phase to prepare sample solutions having a concentration of 5 mg/mL. According to chromatographic peak positions of the dextran standard curve and polysaccharide sample chromatograms, molecular weight distribution ranges of the polysaccharide samples were calculated to be 4.063 kDa to $2.289\times10^2$ kDa, 2.912 kDa to $1.594\times10^2$ kDa, 3.637 kDa to $1.757\times10^2$ kDa, 3.589 kDa to $2.500\times10^2$ kDa, 4.832 kDa to $1.872\times10^2$ kDa, and 2.500 kDa to $1.337\times10^2$kDa, respectively.

**Test Example 4: Evaluation of Effects of Polysaccharide Preparation Method Using Non-Polar Macroporous Adsorption Resins, Including Determination of Decolorization Rate, Protein Removal Rate, and Polysaccharide Retention Rate**

Determination of decolorization rate: Polysaccharides obtained before and after treatment with non-polar macroporous adsorption resins in Examples 1-6 were prepared into 1 mg/mL solutions, centrifuged at 3000 r/min, and filtered. Absorbance was measured at 450 nm. According to Formula (1), decolorization rates achieved by the processes of Examples 1-6 were calculated to be 77.34%, 76.23%, 79.21%, 75.96%, 75.73%, and 76.51%, respectively.

$$\text{Decolorization rate }(100\%)=[(A_{\text{before decolorization}}-A_{\text{after decolorization}})/A_{\text{before decolorization}}]\times100\% \quad (1)$$

Determination of protein removal rate: Protein content was determined using the Coomassie brilliant blue method. Bovine serum albumin standard solutions at concentrations of 0.00, 0.02, 0.04, 0.06, 0.08, 0.10, and 0.12 mg/mL were prepared to construct a standard curve. Polysaccharides obtained before and after treatment with non-polar macroporous adsorption resins in Examples 1-6 were accurately weighed and prepared into 0.2 mg/mL solutions. 3 mL of each sample solution was accurately transferred into a test tube, and 3 mL of Coomassie brilliant blue reagent was added and mixed uniformly. Absorbance was measured at 595 nm. Protein contents were calculated according to the standard curve. According to Formula (2), protein removal rates achieved by the processes of Examples 1-6 were calculated to be 75.84%, 73.74%, 74.65%, 73.19%, 73.23%, and 74.39%, respectively.

Protein removal rate (%)=[(protein content before deproteinization (mg/mL)-protein content after deproteinization (mg/mL))/protein content before deproteinization (mg/mL)]×100%

Determination of polysaccharide retention rate: Polysaccharide content was determined using the phenol-sulfuric acid method. Glucose standard solutions at concentrations of 0.00, 0.02, 0.04, 0.06, 0.08, 0.10, and 0.12 mg/mL were prepared to construct a standard curve. Polysaccharides obtained before and after treatment with non-polar macroporous adsorption resins in Examples 1-6 were accurately weighed and prepared into 0.1 mg/mL solutions. 2 mL of each sample solution was accurately transferred into a test tube, and 2 mL of 6% phenol and 6 mL of concentrated $H_2SO_4$ were sequentially added, followed by mixing. The mixture was allowed to stand for 10 min and then heated in a 100 °C water bath for 20 min. After cooling to room temperature, absorbance was measured at 490 nm. Polysaccharide contents were calculated according to the standard curve. According to Formula (3), polysaccharide retention rates after treatment in Examples 1-6 were calculated to be 70.94%, 74.75%, 71.94%, 78.94%, 76.53%, and 75.83%, respectively.

Polysaccharide retention rate (%)=(polysaccharide content before resin adsorption (mg/mL)/polysaccharide content after resin adsorption (mg/mL))×100%

**Test Example 5: In Vivo Evaluation of Anti-Ulcerative Colitis Activity of polysaccharide from *Hippophae Rhamnoides* Fruit Peel**

Experimental animals: Male Kunming mice, 6-8 weeks old.

Experimental grouping: Mice were randomly divided into seven groups: a normal group (Con), a model group (DSS), treatment groups receiving polysaccharide from *Hippophae rhamnoides* fruit peel obtained in Example 1 (50 mg/kg/d and 100 mg/kg/d), a treatment group receiving polysaccharide from *Hippophae rhamnoides* seed meal obtained in Comparative Example 1 (100 mg/kg/d), a treatment group receiving polysaccharide from *Hippophae rhamnoides* fruits obtained in Comparative Example 2 (100 mg/kg/d), and a positive control group receiving 100 mg/kg/d sulfasalazine (SAZ).

Experimental method: Ulcerative colitis (UC) mice were induced using DSS. After 56 Kunming mice aged 6-8 weeks were acclimation-fed for one week, 8 mice were randomly assigned to a normal group and 48 mice were randomly assigned to a model induction group. From day 1, mice in the normal group were provided with normal drinking water until day 14. Mice in the model induction group were administered 2.5% DSS in drinking water until day 7 to induce a UC model. After successful model establishment, mice in the model induction group were randomly divided into a model group,

treatment groups receiving polysaccharide from *Hippophae rhamnoides* fruit peel obtained in Example 1 (50 mg/kg/d and 100 mg/kg/d), a treatment group receiving polysaccharide from *Hippophae rhamnoides* seed meal obtained in Comparative Example 1 (100 mg/kg/d), a treatment group receiving polysaccharide from *Hippophae rhamnoides* fruits obtained in Comparative Example 2 (100 mg/kg/d), and a positive control group receiving 100 mg/kg/d sulfasalazine, with 8 mice in each group. From day 8 to day 14, mice in the normal group and the model group were administered 0.2 mL of normal saline by gavage once daily; mice in the treatment groups receiving 50 mg/kg/d and 100 mg/kg/d polysaccharide from *Hippophae rhamnoides* fruit peel were administered 0.2 mL of polysaccharide solutions by gavage once daily according to body weight; mice in the treatment group receiving polysaccharide from *Hippophae rhamnoides* seed meal obtained in Comparative Example 1 (100 mg/kg/d) and mice in the treatment group receiving polysaccharide from *Hippophae rhamnoides* fruits obtained in Comparative Example 2 (100 mg/kg/d) were treated in the same manner until day 14; and mice in the positive control group were administered 0.2 mL of a SAZ solution by gavage once daily according to body weight until day 14.

After administration on day 14, mice were fasted for 18 h with free access to water. On day 15, blood was collected from the orbital venous plexus. Blood samples were allowed to stand at room temperature for 30 min and were centrifuged at 4000 r/min and 4 °C for 10 min. Serum was collected and stored at -80 °C. Mice were sacrificed by cervical dislocation and dissected. Colon tissues were excised, colon lengths were measured, and photographs were taken. A 0.5-0.8 cm segment from the distal colon was circumferentially excised, fixed with 4% paraformaldehyde for 24-48 h, dehydrated, embedded in paraffin, and sectioned for subsequent immunohistochemistry (IHC) analysis of colonic lesions and intestinal barrier function. Upper colon tissues were longitudinally opened, intestinal contents were removed, tissues were rinsed with normal saline, residual liquid was removed, intestinal mucosal tissues were scraped using glass slides, and intestinal mucosal tissues were stored at -80 °C for subsequent experiments.

Observation of mouse signs and disease activity index (DAI) scoring: During the experimental period, body weights of mice in each group were measured and recorded every other day, stool characteristics were recorded, and fecal occult blood was detected using a fecal occult blood test kit until day 14. DAI scores were assigned according to Table 1.

Table 1. DAI scoring criteria

| Score | Body weight loss (%) | Stool characteristics | Occult blood |
|---|---|---|---|
| 0 | None | normal | No blue-green color within 3 min |
| 1 | 0-5% | | Blue color appears within 30-60 s |
| 2 | 5-10% | Loose stool | Blue-green color appears immediately |
| 3 | 10-15% | | Dark blue color appears immediately |
| 4 | >15% | Diarrhea | Blackish-brown color appears immediately |

IHC analysis of intestinal barrier function protein expression in UC mice: Tissue sections were baked until paraffin was completely melted, immersed in xylene for 20 min, and then sequentially immersed in absolute ethanol, 95% ethanol, 85% ethanol, 75% ethanol, and distilled water for deparaffinization and rehydration. Antigen retrieval was performed according to a conventional procedure using a citrate antigen retrieval buffer (pH 6.0). After permeabilization with 0.3% Triton, slides were washed with PBS. Tissue sections were placed in a humidified chamber, endogenous peroxidase was inactivated, and slides were washed with PBS. Tissue sections were blocked with goat serum, followed by addition of a ZO-1 antibody dilution or an OCLN antibody dilution and incubation at room temperature for 2 h. After PBS washing, a secondary antibody dilution was added and allowed to react at room temperature for 1 h, followed by PBS washing. DAB solution was added for color development. Cell nuclei were counterstained with hematoxylin. After washing, tissue sections were sequentially dehydrated and cleared in 75% ethanol, 85% ethanol, 95% ethanol, absolute ethanol, and xylene, followed by mounting with a neutral resin. Three mouse tissue sections were randomly selected from each group, and three fields of view were selected from each tissue section (n = 9). Observation and photography were performed under a 200× microscope, and images were analyzed using Image-Pro Plus software.

Determination of inflammatory factors in mouse serum and colon tissues by ELISA: Contents of TNF-$\alpha$ and IL-10 in serum and colon tissues of mice in each group were determined according to procedures provided with a mouse ELISA kit. 20 mg of colonic mucosa was weighed, normal saline was added at a ratio of 1:3, and homogenization was performed using a homogenizer until no obvious tissue fragments remained. Samples were centrifuged at 4000 r/min and 4 °C for 10 min, and supernatants were collected. Contents of TNF-$\alpha$ and IL-10 in colon tissues were determined using a mouse ELISA kit. Serum samples were removed from a -80 °C freezer and thawed at room temperature. Contents of TNF-$\alpha$ and IL-10 in serum were determined according to procedures provided with the mouse ELISA kit.

Statistical analysis: Data were expressed as mean ± standard error (mean ± SE), and homogeneity of variance testing and one-way analysis of variance (One-way ANOVA) or Student's *t*-test were performed.

**Experimental results:**

**Effects of polysaccharide from *Hippophae rhamnoides* fruit peel on body weight and DAI in UC mice:**

During the experimental period, body weights of mice in the normal group continuously increased. In contrast, after DSS induction from day 1 to day 7, body weights of model mice decreased by approximately 10%-20%. From initiation of model induction on day 1 until completion of the experiment on day 14, body weights of mice in the model group decreased by approximately 30%. Administration to treatment groups was initiated on day 8 and continued until day 14. Body weights of DSS-induced UC mice gradually increased. Compared with mice in the model group, mice in the treatment group receiving 50 mg/kg/d of polysaccharide from *Hippophae rhamnoides* fruit peel, mice in the treatment group receiving 100 mg/kg/d of polysaccharide from *Hippophae rhamnoides* fruit peel, and mice in the positive control group exhibited significantly increased body weights, as shown in Table 2. These results indicate that polysaccharide from *Hippophae rhamnoides* fruit peel can inhibit body weight loss in UC mice.

From day 1 of model induction, DAI scores of DSS-induced mice in each group continuously increased. Compared with the DSS group, DAI scores of treatment groups gradually decreased after administration of polysaccharide from *Hippophae rhamnoides* fruit peel, polysaccharide from *Hippophae rhamnoides* seed meal, polysaccharide from *Hippophae rhamnoides* fruits, or SAZ beginning on day 8. After continuous administration until day 14, DAI scores of mice in all treatment groups were significantly lower than DAI scores of mice in the DSS group. Compared with the treatment group receiving polysaccharide from *Hippophae rhamnoides* seed meal at the same dosage and the treatment group receiving polysaccharide from *Hippophae rhamnoides* fruits at the same dosage, the 100 mg/kg/d treatment group receiving polysaccharide from *Hippophae rhamnoides* fruit peel exhibited superior efficacy, as shown in Table 2. The above results demonstrate that administration of polysaccharide from *Hippophae rhamnoides* fruit peel significantly alleviated the severity of DSS-induced ulcerative colitis in mice.

Table 2. Effects of the polysaccharide from *Hippophae rhamnoides* fruit peel on body weight and DAI in UC mice (n=8)

| Group | Body weight on day 14 (g) | DAI on day 14 |
|---|---|---|
| Normal group | 37.81±1.96** | 0** |
| Model group | 24.43±1.53 | 2.33±0.14 |
| Treatment group receiving 50 mg/kg/d of polysaccharide from *Hippophae rhamnoides* fruit peel | 29.24±1.65* | 1.33±0.08* |
| Treatment group receiving 100 mg/kg/d of polysaccharide | 31.38±1.88** | 0.67±0.13** |
| from *Hippophae rhamnoides* fruit peel | | |
| Treatment group receiving 100 mg/kg/d of polysaccharide from *Hippophae rhamnoides* seed meal | 27.11±1.02 | 1.35±0.11* |
| Treatment group receiving 100 mg/kg/d of polysaccharide from *Hippophae rhamnoides* fruits | 29.11±1.62* | 1.34±0.13* |
| Positive control group | 31.61±1.94** | 1.24±0.11* |
| Note: Compared with the model group, * indicates significant difference, *P<0.05; *P<0.01. | | |

**Effects of the polysaccharide from *Hippophae rhamnoides* fruit peel on colon length of UC mice**

Compared with the normal control group, the model group exhibited a 28.79% reduction in colon length. The treatment group receiving polysaccharide from *Hippophae rhamnoides* fruit peel and the positive control group significantly attenuated the colon shortening, showing better efficacy than the treatment group receiving polysaccharide from *Hippophae rhamnoides* seed meal and the treatment group receiving polysaccharide from *Hippophae rhamnoides* fruits. Compared with the model group, treatment with 100 mg/kg/d polysaccharides from *Hippophae rhamnoides* fruit peel and 100 mg/kg/d SAZ increased the colon length of UC mice by 24.68% and 12.02%, respectively, and the treatment effect of 100 mg/kg/d polysaccharides from *Hippophae rhamnoides* was more significant than that of SAZ (see Table 3). The results indicate that treatment with polysaccharide from *Hippophae rhamnoides* fruit peel reduces colon damage in UC mice and exerts a protective effect against DSS-induced colon damage.

Table 3. Effect of polysaccharide from *Hippophae rhamnoides* fruit peel on colon length of UC mice (n=8)

| Group | Colon length of mice (cm) |
|---|---|
| Normal group | 9.37±0.17** |
| Model group | 7.31±0.21 |
| Treatment group receiving 50 mg/kg/d of polysaccharide from *Hippophae rhamnoides* fruit peel | 7.83±0.09 |
| Treatment group receiving 100 mg/kg/d of polysaccharide from *Hippophae rhamnoides* fruit peel | 9.11±0.15** |
| Treatment group receiving 100 mg/kg/d of polysaccharide from *Hippophae rhamnoides* seed meal | 7.82±0.05 |
| Treatment group receiving 100 mg/kg/d of polysaccharide from *Hippophae rhamnoides* fruits | 7.84±0.07 |
| Positive control group | 8.18±0.14* |
| Note: Compared with the model group, * indicates significant difference, *P<0.05; *P<0.01. | |

**Effects of polysaccharide from *Hippophae rhamnoides* fruit peel on intestinal barrier function in UC mice**

The relative expression levels of ZO-1 and OCLN proteins were quantified. Compared with the normal group, the model group exhibited markedly reduced or even absent expression of ZO-1 and OCLN proteins between colonic mucosal epithelial cells, resulting in disruption of tight junction integrity and severe impairment of colonic barrier function. In contrast, treatment with the polysaccharide from *Hippophae rhamnoides* fruit peel significantly increased the expression levels of ZO-1 and OCLN compared with the model group. The therapeutic effect was superior to that of the polysaccharide from *Hippophae rhamnoides* seed meal and the polysaccharide from *Hippophae rhamnoides* fruits. In addition, administration of 100 mg/kg/d of polysaccharide from *Hippophae rhamnoides* fruit peel exhibited a protective effect on the intestinal barrier function of colonic tissue in UC mice comparable to that of the positive control group. The treatment with the polysaccharide from *Hippophae rhamnoides* fruit peel reduced intestinal permeability and alleviated intestinal functional impairment in UC mice, suggesting a significant therapeutic effect of the polysaccharide from *Hippophae rhamnoides* fruit peel against DSS-induced UC (see Table 4).

Table 4. Relative expression levels of ZO-1 and OCLN in colon tissue of UC mice (n=9)

| Group | ZO-1 | OCLN |
|---|---|---|
| Normal group | 1.03 ±0.04** | 1.04±0.04** |
| Model group | 0.49±0.01 | 0.37±0.02 |
| Treatment group receiving 50 mg/kg/d of polysaccharide from *Hippophae rhamnoides* fruit peel | 0.58±0.03 | 0.57±0.05* |
| Treatment group receiving 100 mg/kg/d of polysaccharide from *Hippophae rhamnoides* fruit peel | 0.83±0.05* | 0.78±0.06** |
| Treatment group receiving 100 mg/kg/d of polysaccharide from *Hippophae rhamnoides* seed meal | 0.60±0.05 | 0.53±0.09 |
| Treatment group receiving 100 mg/kg/d of polysaccharide from *Hippophae rhamnoides* fruits | 0.57±0.02 | 0.55±0.01* |
| Positive control group | 0.85±0.03* | 0.79±0.05** |
| Note: Compared with the model group, * indicates significant difference, *P<0.05; *P<0.01. | | |

**Effects of polysaccharide from *Hippophae rhamnoides* fruit peel on TNF-α and IL-10 levels in serum and colon tissue of UC mice:**

Compared with the normal group, the model group exhibited a significant increase in TNF-α levels in colonic tissue of mice, indicating a severe inflammatory response in UC mice. In contrast, the TNF-α levels in the colonic tissues of mice in the treatment groups receiving polysaccharides from *Hippophae rhamnoides* fruit peel, polysaccharides from *Hippophae rhamnoides* seed meal, or polysaccharides from *Hippophae rhamnoides* fruits were significantly lower than

that in the DSS group. Moreover, treatment with 100 mg/kg/d polysaccharides from *Hippophae rhamnoides* fruit peel exhibited superior efficacy compared with the same dose of polysaccharides from *Hippophae rhamnoides* seed cake, polysaccharides from *Hippophae rhamnoides* fruits, and SAZ. Similarly, compared with the normal group, DSS induction significantly increased TNF-α levels in the serum of UC mice. Treatment with polysaccharides from *Hippophae rhamnoides* fruit peel effectively reduced TNF-α levels in the serum of UC mice, and the effect of 100 mg/kg/d polysaccharides from *Hippophae rhamnoides* fruit peel was comparable to that of SAZ and superior to that of the same dose of polysaccharides from *Hippophae rhamnoides* seed meal and polysaccharides from *Hippophae rhamnoides* fruits.

In the serum samples of mice, compared with the normal group, IL-10 levels in the model group were significantly decreased. Compared with the model group, administration of 50 mg/kg/d or 100 mg/kg/d of polysaccharides from *Hippophae rhamnoides* fruit peel promoted IL-10 secretion, resulting in a significant increase in the concentration. In addition, the effect of administration of 100 mg/kg/d of polysaccharides from *Hippophae rhamnoides* fruit peel in promoting IL-10 secretion was superior to that of the treatment group receiving polysaccharides from *Hippophae rhamnoides* seed meal and the treatment group receiving polysaccharides from *Hippophae rhamnoides* fruit, and was comparable to that of the positive control drug sulfasalazine (SAZ). Similar to the results obtained from the serum samples, DSS induction significantly reduced IL-10 levels in the colonic tissues of model mice. However, administration of 100 mg/kg/d of polysaccharides from *Hippophae rhamnoides* fruit peel or SAZ significantly increased the level of this anti-inflammatory factor, with efficacy superior to that of the treatment group receiving polysaccharides from *Hippophae rhamnoides* seed meal and the treatment group receiving polysaccharides from *Hippophae rhamnoides* fruits. These results indicate that polysaccharide from *Hippophae rhamnoides* fruit peel exerts anti-inflammatory effects by promoting the secretion of anti-inflammatory factors and inhibiting inflammatory responses in DSS-induced UC mice, thereby achieving a therapeutic effect against UC. The specific results are shown in Table 5.

Table 5. Effects of polysaccharide from *Hippophae rhamnoides* fruit peel on TNF-α and IL-10 levels in serum and colon tissue of UC mice (n=5)

| Group | TNF-α level (pg/mL) | | IL-10 level (pg/mL) | |
|---|---|---|---|---|
| | Serum | Colonic tissue | Serum | Colonic tissue |
| Normal group | 35.98±3.16** | 48.46±6.41** | 88.43±15.32** | 108.59±19.24** |
| Model group | 88.46±11.77 | 136.81±29.53 | 30.15±4.32 | 45.72±5.82 |
| Treatment group receiving | 61.93±8.58* | 79.42±12.53* | 47.30±4.69* | 49.83±5.73* |
| 50 mg/kg/d of polysaccharide from *Hippophae rhamnoides* fruit peel | | | | |
| Treatment group receiving 100 mg/kg/d of polysaccharide from *Hippophae rhamnoides* fruit peel | 49.26±5.31** | 56.97±7.32** | 90.47±16.31** | 112.43±25.81** |
| Treatment group receiving 100 mg/kg/d of polysaccharide from *Hippophae rhamnoides* seed meal | 62.47±7.93* | 77.12±12.39* | 48.27±4.52* | 51.03±2.77* |
| Treatment group receiving 100 mg/kg/d of polysaccharide from *Hippophae rhamnoides* fruits | 59.62±7.42** | 76.12±11.24** | 49.81±5.10* | 53.66±3.05* |
| Positive control group | 52.40±5.94** | 58.33±7.45** | 87.85±16.02** | 114.82±25.94** |
| Note: Compared with the model group, * indicates significant difference, *P<0.05; *P<0.01. | | | | |

**Example 6. Evaluation of the Effects of Polysaccharides from *Hippophae rhamnoides* Fruit Peel on Functional Constipation and Functional Dyspepsia in Rats**

Experimental animals: Male SD rats, weighing 120-150 g.

**Experiment 1: Effects of polysaccharides from *Hippophae rhamnoides* fruit peel on defecation in rats**

Experimental method: A constipation model was established by oral administration of loperamide hydrochloride. 30 male SD rats were acclimation-fed for one week. All rats were randomly divided into six groups: a normal group, a model group, three dose groups of polysaccharides from *Hippophae rhamnoides* fruit peel (a high dose of 50 mg/kg/d, a medium

dose of 25 mg/kg/d, a low dose of 12.5 mg/kg/d), and a positive control group receiving Ma-Ren pills at 0.6 g/kg/d, with 5 rats per group. The intervention period lasted 7 days. Rats in the three dose groups of polysaccharides from *Hippophae rhamnoides* fruit peel were orally administered 1 mL of the corresponding solution according to body weight. Rats in the positive control group were orally administered 1 mL of Ma-Ren pills solution (0.6 g/kg/d) according to body weight. Rats in the normal group and the model group were orally administered an equal volume of normal saline. After the intervention period, rats were fasted for 16 h with free access to water. The constipation model was then induced: the normal group was orally administered 1 mL of normal saline, while the model group, the positive control group, and the treatment groups receiving polysaccharides from *Hippophae rhamnoides* fruit peel were orally administered loperamide hydrochloride at 3 mg/kg in 1 mL. 0.5 h later, the normal group and model group were orally administered 10 mL/kg of India ink, while the positive control group and the dose groups were orally administered 10 mL/kg of India ink containing the corresponding dose of test substance. The time to first black stool, the number of black fecal pellets within 6 h, and the fecal weight were recorded.

**Experiment 2: Effects of Polysaccharides from *Hippophae rhamnoides* Fruit Peel on Small Intestinal Motility in Rats**

Experimental method: A constipation model was established by oral administration of loperamide hydrochloride. 30 male SD rats were acclimation-fed for one week. All rats were randomly divided into six groups: a normal group, a model group, three dose groups of polysaccharides from *Hippophae rhamnoides* fruit peel (a high dose of 50 mg/kg/d, a medium dose of 25 mg/kg/d, a low dose of 12.5 mg/kg/d), and a positive control group receiving Ma-Ren pills at 0.6 g/kg/d, with 5 rats per group. The intervention period lasted 7 days. Rats in the three dose groups of polysaccharides from *Hippophae rhamnoides* fruit peel were orally administered 1 mL of the corresponding solution according to body weight. Rats in the positive control group were orally administered 1 mL of Ma-Ren pills solution (0.6 g/kg/d) according to body weight. Rats in the normal group and the model group were orally administered an equal volume of normal saline. After the intervention period, rats were fasted for 16 h with free access to water. The constipation model was then conducted: the normal group was orally administered 1 mL of normal saline, while the model group, the positive control group, and the treatment groups receiving polysaccharides from *Hippophae rhamnoides* fruit peel were orally administered loperamide hydrochloride at 3 mg/kg in 1 mL. 0.5 h later, the normal group and model group were orally administered 10 mL/kg of India ink, while the positive control group and the dose groups were orally administered 10 mL/kg of India ink containing the corresponding dose of test substance. 25 min after administration, rats were immediately euthanized by cervical dislocation. The abdominal cavity was opened, and the mesentery was separated. The small intestine from the pylorus to the ileocecal junction was gently straightened, and the total length of the small intestine was measured. The distance from the pylorus to the leading edge of the India ink was recorded as the "ink propulsion length".

Small intestinal propulsion rate calculation: small intestinal propulsion rate (%)=ink propulsion length (cm)/total small intestinal length (cm)×100%

**Experiment 3: Effects of Polysaccharides from *Hippophae rhamnoides* Fruit Peel on Digestive Function in Rats**

Experimental method:20 male SD rats were acclimation-fed for one week. All rats were randomly divided into four groups: a normal group, and three dose groups of polysaccharides from *Hippophae rhamnoides* fruit peel (a high dose of 50 mg/kg/d, a medium dose of 25 mg/kg/d, a low dose of 12.5 mg/kg/d), with 5 rats per group. The intervention period lasted 30 days. Before the end of the experiment, rats were fasted for 24 h with free access to water. Gastric juice was collected from each rat using pyloric ligation under isoflurane anesthesia, and the gastric juice volume per unit time was measured. Glass capillaries with an inner diameter of 1 mm and length of 100 mm were filled with fresh egg white and exposed to hot steam for 2 min to coagulate the protein, thereby preparing protein tubes. The tubes were stored at 4 °C and cut to a length of 20 mm prior to use. 1 mL of gastric juice was added to a 50 mL conical flask containing 15 mL of 0.05 mol/L hydrochloric acid solution and shaken well, and two freshly prepared protein tubes were placed into each flask. After sealing, the flasks were incubated at 37 °C for 24 h. The protein tubes were then removed, and the length of the transparent portion at both ends of each tube was measured using a vernier caliper. The average of four measured ends was calculated. Gastric pepsin activity and gastric pepsin output were calculated as follows:

$$\text{Gastric pepsin activity } (\mu/\text{mL}) = \text{average transparent length of four ends}^2 \times 16$$

Gastric pepsin output (μ/h)=gastric pepsin activity×gastric juice volume per hour

Statistical analysis: Data were expressed as mean ± standard error (mean ± SE), and homogeneity of variance

testing and one-way analysis of variance (One-way ANOVA) or Student's *t*-test were performed.

Experimental results: Compared with the model group, rats treated with polysaccharides from *Hippophae rhamnoides* fruit peel exhibit significantly increased number of fecal pellets, fecal weight, fecal moisture content, and small intestinal propulsion rate within 6 h, and the time to first defecation was significantly shortened, as shown in Tables 6 and 7. After intervention with polysaccharides from *Hippophae rhamnoides* fruit peel, the gastric juice volume per hour was significantly increased, gastric pepsin activity was significantly elevated, and gastric pepsin output was significantly higher, as shown in Table 8. These results indicate that polysaccharides from *Hippophae rhamnoides* fruit peel promote digestion and improve functional constipation in rats.

Table 6. Effects of polysaccharides from *Hippophae rhamnoides* fruit peel on defecation function in constipated rats (n=5)

| Group | Time to first defecation (min) | Number of fecal pellets within 6 h | Fecal weight (mg) | Fecal moisture content (%) |
|---|---|---|---|---|
| Normal group | 72.43 ±14.22** | 17.55 ±2.91** | 592.50 ±21.24** | 65.34±12.41* |
| Model group | 128.93±21.94 | 8.62±1.01 | 268.24±20.65 | 52.09±14.20 |
| Treatment group receiving 12.5 mg/kg/d of polysaccharide from | 79.34±13.06* | 12.72±3.83* | 452.47 ±19.35** | 59.03±13.01 |
| *Hippophae rhamnoides* fruit peel | | | | |
| Treatment group receiving 25 mg/kg/d of polysaccharide from *Hippophae rhamnoides* fruit peel | 73.34±7.99** | 15.20 ±2.63** | 508.90 ±22.35** | 62.03±11.02 |
| Treatment group receiving 50 mg/kg/d of polysaccharide from *Hippophae rhamnoides* fruit peel | 68.34±7.37** | 18.20 ±2.63** | 573.74 ±24.41** | 65.21±9.26* |
| Positive control group | 69.82±11.09** | 17.86 ±3.05** | 582.60 ±20.26** | 65.28±11.41* |
| Note: Compared with the model group, * indicates significant difference, *P<0.05; *P<0.01. | | | | |

Table 7. Effects of polysaccharides from *Hippophae rhamnoides* fruit peel on small intestinal motility in constipated rats (n=5)

| Group | Small intestinal propulsion rate (%) |
|---|---|
| Normal group | 72.03±8.94** |
| Model group | 49.86±8.56 |
| Treatment group receiving 12.5 mg/kg/d of polysaccharide from *Hippophae rhamnoides* fruit peel | 55.32±7.63* |
| Treatment group receiving 25 mg/kg/d of polysaccharide from *Hippophae rhamnoides* fruit peel | 69.28±6.25* |
| Treatment group receiving 50 mg/kg/d of polysaccharide from *Hippophae rhamnoides* fruit peel | 71.88±5.25** |
| Positive control group | 72.21±4.20** |
| Note: Compared with the model group, * indicates significant difference, *P<0.05; *P<0.01. | |

Table 8. Effects of polysaccharides from *Hippophae rhamnoides* fruit peel on gastric juice secretion and pepsin in rats (n=5)

| Group | Gastric juice volume (mL/h) | Gastric pepsin activity (μ/mL) | Gastric pepsin output (m/h) |
|---|---|---|---|
| Normal group | 0.95±0.23 | 83.65±11.09 | 142.43±15.09 |

(continued)

| Group | Gastric juice volume (mL/h) | Gastric pepsin activity (μ/mL) | Gastric pepsin output (m/h) |
|---|---|---|---|
| Treatment group receiving 12.5 mg/kg/d of polysaccharide from *Hippophae rhamnoides* fruit peel | 1.27±0.16* | 97.12±11.47* | 159.72±15.85* |
| Treatment group receiving 25 mg/kg/d of polysaccharide from *Hippophae* | 1.29±0.23* | 104.30±12.52* | 161.53±19.09* |
| *rhamnoides* fruit peel | | | |
| Treatment group receiving 50 mg/kg/d of polysaccharide from *Hippophae rhamnoides* fruit peel | 1.34±0.21* | 116.59±13.20** | 164.43±23.60* |
| Note: Compared with the normal group, * indicates significant difference, *P<0.05; *P<0.01. | | | |

**Example 7: Evaluation of the Activity of Polysaccharides from *Hippophae rhamnoides* Fruit Peel on Gut Microbiota**

Experimental animals: Male BALB/C mice, 18-22 g.

Experimental method: After acclimation feeding, 40 mice were randomly divided into a normal group (n=8) and a model induction group (n=32). From day 1 to day 10, the model induction group was orally administered cefixime at 375 mg/kg/d, and the normal group was orally administered an equal volume of normal saline. After 10 days of modeling, upon successful establishment of the model, the model induction group was subdivided into a model group and treatment groups receiving polysaccharides from *Hippophae rhamnoides* fruit peel at high dose (100 mg/kg), medium dose (50 mg/kg), or low dose (25 mg/kg). From day 11 to day 20, the treatment groups were administered polysaccharides from *Hippophae rhamnoides* fruit peel for treatment, while the normal control group was orally administered an equal volume of normal saline. Fecal samples were collected throughout the experimental period. At 24 h after the final administration, ileal and cecal contents were collected under sterile conditions, serially diluted to $10^{-9}$, and inoculated onto selective media for gut microbiota analysis. Blood samples were collected, and mice were anesthetized and sacrificed. Tissues were harvested and stored at -80 °C.

Statistical analysis: Data were expressed as mean ± standard error (mean ± SE), and homogeneity of variance testing and one-way analysis of variance (One-way ANOVA) or Student's *t*-test were performed.

Experimental results: Treatment with 50 mg/kg/d and 100 mg/kg/d of polysaccharides from *Hippophae rhamnoides* fruit peel can gradually restore body weight in mice with gut microbiota dysbiosis (Table 9). Each treatment group receiving polysaccharides from *Hippophae rhamnoides* fruit peel can restore intestinal bacterial counts in mice with gut microbiota dysbiosis to normal levels, showing significant differences compared with the model group (Table 10).

Table 9. Effect of polysaccharides from *Hippophae rhamnoides* fruit peel on body weight in gut microbiota dysbiosis model mice (n=8)

| Group | Mouse body weight (g) | | | | |
|---|---|---|---|---|---|
| | 0 d | 5 d | 10 d | 15 d | 20 d |
| Normal group | 19.93±0.11 | 25.15±0.31 | 26.58±0.28* | 27.88±0.17** | 29.31±0.22** |
| Model group | 18.45±0.21 | 26.36±0.53 | 27.58±0.28 | 30.03±0.28 | 31.26±0.23 |
| Treatment group receiving 25 mg/kg/d of polysaccharide from *Hippophae rhamnoides* fruit peel | 18.77±0.19 | 25.79±0.47 | 27.63±0.28 | 28.93±0.38 | 29.93±0.15 |
| Treatment group receiving 50 mg/kg/d of polysaccharide from *Hippophae rhamnoides* fruit peel | 18.52±0.13 | 26.16±0.81 | 27.49±0.28 | 28.51±0.16* | 29.67±0.41* |
| Treatment group receiving 100 mg/kg/d of polysaccharide from *Hippophae rhamnoides* fruit peel | 19.32±0.12 | 25.58±0.62 | 26.91±0.28 | 28.01±0.24* | 29.33±0.17** |
| Note: Compared with the model group, * indicates significant difference, *P<0.05; *P<0.01. | | | | | |

Table 10. Effect of polysaccharides from *Hippophae rhamnoides* fruit peel on normal gut microbiota counts in gut microbiota dysbiosis model mice (n=8)

| Group | Post-treatment gut microbiota counts (Log cfu.mL$^{-1}$) | | | |
|---|---|---|---|---|
| | Total colonies | *Escherichia coli* | *Lactobacillus* | *Bifidobacterium* |
| Normal group | 9.31±0.51** | 11.83±0.82* | 12.02±0.72** | 11.76±1.22** |
| Model group | 7.22±0.47 | 10.26±0.76 | 10.18±0.65 | 9.20±0.62 |
| Treatment group receiving 25 mg/kg/d of polysaccharide from *Hippophae rhamnoides* fruit peel | 8.25±0.49* | 11.76±0.82* | 12.52±1.03* | 11.63±1.33* |
| Treatment group receiving 50 mg/kg/d of polysaccharide from *Hippophae rhamnoides* fruit peel | 8.37±0.50* | 11.83±1.02* | 12.62±1.03* | 11.75±1.51* |
| Treatment group receiving 100 mg/kg/d of polysaccharide from *Hippophae rhamnoides* fruit peel | 8.49±0.30* | 11.95±1.14* | 12.95±1.01** | 11.93±1.07** |
| Note: Compared with the model group, * indicates significant difference, *P<0.05; *P<0.01. | | | | |

Although the specific embodiments of the present application have been described in detail, these embodiments should not be construed as limiting the scope of protection of the present application. Various modifications and variations that can be made within the scope described in the claims by those skilled in the art without creative efforts still belong to the protection scope of the present application.

## Claims

1. A polysaccharide from *Hippophae rhamnoides* fruit peel, comprising the following monosaccharide components: rhamnose, mannose, galactose, glucose, galacturonic acid, and arabinose in a molar ratio of 1:2.03-2.63:2.65-3.17:3.20-3.97:4.01-7.33:7.95-11.67.

2. The polysaccharide from *Hippophae rhamnoides* fruit peel according to claim 1, wherein a mass percentage content of neutral sugars in the polysaccharide from *Hippophae rhamnoides* fruit peel is 82.73%-85.31%, and a molecular weight distribution range of the polysaccharide from *Hippophae rhamnoides* fruit peel is 2.5 kDa to $2.5\times10^2$ kDa.

3. The polysaccharide from *Hippophae rhamnoides* fruit peel according to claim 1, wherein a mass percentage content of uronic acid in the polysaccharide from *Hippophae rhamnoides* fruit peel is 16.90%-23.78%.

4. The polysaccharide from *Hippophae rhamnoides* fruit peel according to claim 1, wherein the molar ratio of the rhamnose, the mannose, the galactose, the glucose, the galacturonic acid, and the arabinose is 1:2.10-2.63:2.65-3.17:3.20-3.97:4.10-6.35:9.60-11.30.

5. A preparation method for the polysaccharide from *Hippophae rhamnoides* fruit peel, comprising the following steps:

(1) defatting: adding dried *Hippophae rhamnoides* fruit peel to an organic solvent for extraction by a reflux method, a maceration method, or a percolation method, discarding an extract, and obtaining defatted *Hippophae rhamnoides* fruit peel residue;
(2) extraction: drying the defatted *Hippophae rhamnoides* fruit peel residue, soaking the defatted *Hippophae rhamnoides* fruit peel residue in water, subsequently performing reflux extraction, and filtering to obtain an extract solution;
(3) concentration: concentrating the extract solution and cooling to obtain a concentrated solution;
(4) alcohol precipitation: adding absolute ethanol to the concentrated solution, or adding the concentrated solution to a high-concentration aqueous ethanol solution, allowing the resulting mixture to stand, and collecting a precipitate to obtain a crude polysaccharide from *Hippophae rhamnoides* fruit peel; and
(5) purification: dissolving the crude polysaccharide from *Hippophae rhamnoides* fruit peel in water, adding the

resulting solution to non-polar macroporous adsorption resin D101, D312, X-5, or HP-20, performing elution, and concentrating and drying an eluate to obtain the polysaccharide from *Hippophae rhamnoides* fruit peel.

**6.** The preparation method for the polysaccharide from *Hippophae rhamnoides* fruit peel according to claim 5, comprising the following steps:

(1) defatting: drying *Hippophae rhamnoides* fruit peel, adding an organic solvent at a solid-to-liquid ratio of 1:5-10 (kg/L) for reflux extraction, wherein the extraction is performed for 2-3 h 1-3 times, filtering, and discarding an extract solution to obtain defatted *Hippophae rhamnoides* fruit peel residue;
(2) extraction: drying the defatted *Hippophae rhamnoides* fruit peel residue, adding water at a solid-to-liquid ratio of 1:6-10 (kg/L) to soak the residue for several hours, subsequently performing reflux extraction, wherein the extraction is performed for 2-3 h 2-3 times, filtering, and combining water extracts;
(3) concentration: concentrating the water extracts to 1/50-1/4 of an original volume and cooling to room temperature to obtain a concentrated solution;
(4) alcohol precipitation: adding absolute ethanol to the concentrated solution under stirring such that a volume concentration of ethanol is 60%-80%, or adding the concentrated solution to a high-concentration aqueous ethanol solution such that a mass percentage content of ethanol in the concentrated solution is 60%-80%, allowing the resulting mixture to stand at 4-25 °C for 6-24 h, discarding a supernatant, and collecting a precipitate to obtain a crude polysaccharide from *Hippophae rhamnoides* fruit peel; and
(5) purification: performing decolorization and protein-removal treatment on the crude polysaccharide from *Hippophae rhamnoides* fruit peel using non-polar macroporous adsorption resin D101, D312, X-5, or HP-20; dissolving the crude polysaccharide from *Hippophae rhamnoides* fruit peel in water, slowly adding a resulting solution to the non-polar macroporous adsorption resin, eluting with water, collecting an eluate, concentrating the eluate to 1/80-1/2 of an original volume, and drying to obtain the polysaccharide from *Hippophae rhamnoides* fruit peel.

**7.** The preparation method for the polysaccharide from *Hippophae rhamnoides* fruit peel according to claim 6, wherein

in step (1), the organic solvent is ether, petroleum ether, ethyl acetate, an aqueous methanol solution containing 75% to 100% methanol by mass, or an aqueous ethanol solution containing 75% to 100% ethanol by mass;
in step (2), a soaking time after addition of water to the defatted *Hippophae rhamnoides* fruit peel residue is 3-24 h;
in step (4), the high-concentration aqueous ethanol solution contains 80% to 100% ethanol by mass;
in step (5), the drying is spray drying, reduced-pressure drying, or freeze drying;
in step (5), the non-polar macroporous adsorption resin is D101 macroporous adsorption resin;
in step (5), a mass concentration of a loading solution of the crude polysaccharide from *Hippophae rhamnoides* fruit peel is 0.005-0.03 kg/L, a loading amount of polysaccharide/resin is 0.01-0.15 kg/kg, a loading flow rate is 1-5 bed volumes (BV)/h, and standing is performed for 12-24 h after completion of loading; and
in step (5), the elution is performed using water, an elution flow rate is 1-5 BV/h, and an elution volume is 1-10 BV.

**8.** Use of the polysaccharide from *Hippophae rhamnoides* fruit peel according to claim 1 or the polysaccharide from *Hippophae rhamnoides* fruit peel prepared by the preparation method according to claim 5 in preparation of a medicament or a functional health product for treating an intestinal functional disease.

**9.** The use according to claim 8, wherein the intestinal functional disease comprises one or more of ulcerative colitis, constipation, dyspepsia, and gut microbiota dysbiosis.

**10.** A composition, comprising the polysaccharide from *Hippophae rhamnoides* fruit peel according to claim 1 or the polysaccharide from *Hippophae rhamnoides* fruit peel prepared by the preparation method according to claim 5, and a pharmaceutically acceptable excipient.

**INTERNATIONAL SEARCH REPORT**

International application No.
**PCT/CN2024/124057**

**A. CLASSIFICATION OF SUBJECT MATTER**

C08B37/00(2006.01)i； A61K131/00(2006.01)i； A61K31/715(2006.01)i

According to International Patent Classification (IPC) or to both national classification and IPC

**B. FIELDS SEARCHED**

Minimum documentation searched (classification system followed by classification symbols)

IPC： C08B37/-； A61K131/-； A61K31/-

Documentation searched other than minimum documentation to the extent that such documents are included in the fields searched

Electronic data base consulted during the international search (name of data base and, where practicable, search terms used)

CNTXT, WPABSC, ENTXT, VEN, CNKI: 沙棘, 多糖, 果皮, 鼠李糖, 甘露糖, 半乳糖, 葡萄糖, 半乳糖醛酸, 阿拉伯糖, sea buckthorn, polysaccharide, pericarp, rhamnose, mannose, galactose, glucose, galacturonic acid, arabinose

**C. DOCUMENTS CONSIDERED TO BE RELEVANT**

| Category* | Citation of document, with indication, where appropriate, of the relevant passages | Relevant to claim No. |
|---|---|---|
| PX | CN 117683150 A (SHAANXI HAIXIANG PLATEAU BIOLOGICAL TECHNOLOGY CO., LTD.) 12 March 2024 (2024-03-12)<br>claims 1-9 | 1-10 |
| Y | 王昕旭 (WANG, Xinxu). "沙棘多糖对DSS 诱导的小鼠溃疡性结肠炎调控机制研究 (Non-official translation: Research on the Regulatory Mechanism of Seabuckthorn Polysaccharide on DSS-induced Ulcerative Colitis in Mice)"<br>*中国优秀硕士学位论文全文数据库 医药卫生科技辑 (Medicine and Health Sciences, China Master's Theses Full-text Database),* No. 01, 15 January 2020 (2020-01-15),<br>page 4, paragraph 1 | 1-10 |
| Y | 关奇等 (GUAN, Qi et al.). "沙棘果皮, 叶中多糖的提取及其抑菌作用研究 (Non-official translation: Study on the Extraction of Polysaccharides from Seabuckthorn Peel and Leaves and Their Antibacterial Effects)"<br>*国际沙棘研究与开发 (The Global Seabuckthorn Research and Development),*<br>Vol. 3, No. 2, 30 June 2005 (2005-06-30), pages 17-20<br>section 2.1 | 1-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **19 December 2024** | **31 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

INTERNATIONAL SEARCH REPORT

International application No.
**PCT/CN2024/124057**

**C. DOCUMENTS CONSIDERED TO BE RELEVANT**

| Category* | Citation of document, with indication, where appropriate, of the relevant passages | Relevant to claim No. |
|---|---|---|
| Y | CN 110003354 A (JILIN INSTITUTE OF CHEMICAL TECHNOLOGY) 12 July 2019 (2019-07-12)<br>description, paragraph 34, table 5 | 1-10 |
| Y | 钟运翠等 (ZHONG, Yuncui et al.). "大孔树脂纯化沙棘果渣多糖的研究 (Non-official translation: Study on Purification of Seabuckthorn Pomace Polysaccharides by Macroporous Resin)"<br>*食品工业科技 (Science and Technology of Food Industry),*<br>Vol. 30, No. 10, 31 October 2009 (2009-10-31), pages 230-236<br>abstract | 5-10 |
| A | CN 115028753 A (HARBIN UNIVERSITY OF COMMERCE) 09 September 2022 (2022-09-09)<br>description, paragraphs 5-17 | 1-10 |
| A | US 5637301 A (NESTEC S.A.) 10 June 1997 (1997-06-10)<br>description, column 2, lines 1-21 | 1-10 |

Form PCT/ISA/210 (second sheet) (July 2022)

**INTERNATIONAL SEARCH REPORT**
**Information on patent family members**

International application No.
**PCT/CN2024/124057**

| Patent document cited in search report | Publication date (day/month/year) | Patent family member(s) | Publication date (day/month/year) |
|---|---|---|---|
| CN 117683150 A | 12 March 2024 | None | |
| CN 110003354 A | 12 July 2019 | CN 110003354 B | 05 April 2022 |
| CN 115028753 A | 09 September 2022 | None | |
| US 5637301 A | 10 June 1997 | GR 3031771 T3 | 29 February 2000 |
| | | EP 0657108 A1 | 14 June 1995 |
| | | EP 0657108 B1 | 08 September 1999 |
| | | KR 950016600 A | 20 July 1995 |
| | | KR 100202072 B1 | 15 June 1999 |
| | | RU 94042247 A | 20 February 1997 |
| | | RU 2136193 C1 | 10 September 1999 |
| | | AU 7897194 A | 22 June 1995 |
| | | AU 676947 B2 | 27 March 1997 |
| | | ES 2138026 T3 | 01 January 2000 |
| | | ATE 184158 T1 | 15 September 1999 |
| | | ZA 949300 B | 07 August 1995 |
| | | DE 69420522 D1 | 14 October 1999 |
| | | DE 69420522 T2 | 23 December 1999 |
| | | ZA 9409300 B | 07 August 1995 |
| | | BR 9404937 A | 08 August 1995 |
| | | JPH 07203930 A | 08 August 1995 |
| | | JP 2909398 B2 | 23 June 1999 |
| | | US 5665361 A | 09 September 1997 |
| | | DK 0657108 T3 | 07 February 2000 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 115028753 A
- CN 110790848 A
- CN 103992299 A


### Non-patent literature cited in the description


- **WANG XINXU**. Regulatory Mechanism of Seabuckthorn Polysaccharide on DSS-Induced Ulcerative Colitis in Mice[D. Inner Mongolia Agricultural University, 2019